# EUROPEAN PATENT APPLICATION

(11) **EP 3 441 113 A1**
(43) Date of publication of application: **13.02.2019**
(21) Application number: 17779064.9
(22) Date of filing: 31.03.2017
(51) Int. Cl.: A61N 2/04, A61G 13/00

(54) **TRANSCRANIAL MAGNETIC STIMULATION SYSTEM, AND POSITIONING ASSISTANCE METHOD, AND PROGRAM**

(30) Priority: 06.04.2016 JP 2016076361
(71) Applicant: Teijin Pharma Limited, Tokyo 100-0013 (JP); Osaka University, Suita-shi, Osaka 565-0871 (JP)
(72) Inventor: SAITOH Youichi, Suita-shi Osaka 565-0871 (JP); HOSOMI Koichi, Suita-shi Osaka 565-0871 (JP); GOTO Yuko, Suita-shi Osaka 565-0871 (JP); SHIMIZU Takeshi, Suita-shi Osaka 565-0871 (JP); MIYAJIMA Kazumoto, Tokyo 100-0013 (JP); FUJIMOTO Katsushi, Tokyo 100-0013 (JP); NAKAMURA Hitoshi, Tokyo 100-0013 (JP); CHIGIRA Junko, Tokyo 100-0013 (JP)
(74) Representative: Hoffmann Eitle
(86) International application number: PCT/JP2017/013717
(87) International publication number: WO 2017/175685

(57) **Abstract**

The present invention is directed to rapid positioning assistance for a mechanical section of a transcranial magnetic stimulation system. This transcranial magnetic stimulation system comprises a coil section 11 that is in contact with or in the vicinity of the head of the subject, and a mechanical section 30 that supports the coil section 11. The mechanical section 30 includes a plurality of adjustment mechanisms that adjust position of a plurality of movable members, and determines position and attitude of the coil section 11 by adjustment using the plurality of adjustment mechanisms. Receiving means receives desired change for at least one of position and attitude of the coil section 11. Guidance means provides guidance of an adjustment procedure for at least one of the plurality of adjustment mechanisms so as to realize adjustment corresponding to the desired change that has been received.

## Description

### Technical Field

The present invention relates to a transcranial magnetic stimulation system and positioning assistance method, and in more detail relates to technology to assist in position of a mechanical section which a transcranial magnetic stimulation system contains.

### Background Art

Conventionally, a transcranial magnetic stimulation system is known. This system is a system that provides magnetic stimulation noninvasively to a subject's brain via the skull, for the purpose of subject pain relief, examination and treatment of depression or the like, and analysis etc. relating to magnetic stimulation to the brain (refer, for example, to patent publication 1).

Magnetic stimulation involves arranging a magnetic stimulation coil (hereafter simply referred to as a coil) close to a stimulation region of a subject's head, causing pulse current to flow in this coil, and generating induced current in a region that constitutes the target of stimulation (hereafter referred to as stimulation region) using a variable magnetic field. Therefore, in this system there is generally provided a mechanical section that can support the coil and hold the coil at an arbitrary position and attitude (orientation). Also, this mechanical section includes a plurality of mechanisms for adjusting positions of a plurality of movable members, and position of the coil is accurately determined by adjusting positions of the movable members using these mechanisms.

On the other hand, proper position and attitude of the coil differs depending on the purpose, and for each subject. For example, in a case where the purpose is pain relief, a stimulation region is constituted by an anatomical region called a motor area within the brain corresponding to a pain area. Also, to provide magnetic stimulation more efficiently to such an anatomical region it is necessary to determine orientation of magnetic stimulation taking into consideration shape of the brain, and shape of the subject's skull including cerebral sulcus etc., but skull and brain shapes differ significantly between individuals. A system operator therefore adjusts position and attitude of the coil every time, in accordance with the purpose of stimulation and the subject.

### Citation List

### Patent Literature

[Patent publication 1] Japanese patent No. 5453325

### SUMMARY OF THE INVENTION

### Problems To Be Solved By The Invention

However, in the mechanical section of the transcranial magnetic stimulation system, movement of the movable members becomes complicated for various reasons. For example, since the object to which magnetic stimulation is applied is the head of a person, some of the movable members of the system are often configured so that the coil moves along a curved line corresponding to the head surface, moves in a direction that faces the center of the head, and rotates with that direction as an axis. In particular, operation of each movable member in order to realize movement of the coil so as to move along a curved line corresponding to the head surface is extremely complicated. Accordingly there are cases where it is difficult for the system operator to rapidly adjust the coil to a desired position and attitude.

Because of this type of situation, there is a demand for technology which can assist with rapid positioning of a mechanical section of a transcranial magnetic stimulation system.

### Means For Solving The Problems

The invention of a first aspect is directed to
a transcranial magnetic stimulation system, comprising
a coil section that comes in to contact with or approaches a head of a subject,
a mechanical section, comprising a plurality of adjustment mechanisms that adjust position of a plurality of movable members, that determines position and attitude of the coil section by adjustment using the plurality of adjustment mechanisms,
receiving means that receives desired change for at least one of position and attitude of the coil section, and
guidance means that provides guidance of an adjustment procedure for at least one of the plurality of adjustment mechanisms so as to realize the desired change that has been received.

The invention of a second aspect is directed to the transcranial magnetic stimulation system of the first aspect, wherein
the receiving means receives input of contact state of the coil section on the head, as the desired change.

The invention of a third aspect is directed to the transcranial magnetic stimulation system of the second aspect, wherein
the contact state is a one-side contact state where only a part, of an outer edge of the coil section, contacts the head.

The invention of a fourth aspect is directed to the transcranial magnetic stimulation system of the third aspect, wherein
the receiving means has choice of a plurality of types of one-side contact state having respectively different states of contact of the outer edge section with the head, and a single one-side contact state selected by an operator is input as the contact state.

The invention of a fifth aspect is directed to the transcranial magnetic stimulation system of the second aspect, where contact state includes an adhered state of the coil section to the head.

The invention of a sixth aspect is directed to the transcranial magnetic stimulation system of the first aspect, wherein the receiving means receives a given amount of change for at least one of position and attitude of the coil section as the desired change.

The invention of a seventh aspect is directed to the transcranial magnetic stimulation system of the sixth aspect, wherein the relative given amount of change is given amount of movement in a given direction, or rotation through a given angular amount for a given rotational direction.

The invention of an eighth aspect is directed to the transcranial magnetic stimulation system of the seventh aspect, wherein the receiving means has choice of a plurality of respectively different types of change that differ in terms of combination of the given direction and given movement amount, or combination of the given rotation direction and angular amount, and one change is selected by the operator as the desired change.

The invention of a ninth aspect is directed to the transcranial magnetic stimulation system of any one of the first to eighth aspects, further provided with acquisition means that acquires position information representing position of the plurality of movable members at the current time, and wherein the guidance means performs guidance of the adjustment procedure using position information that has been acquired by the acquisition means.

The invention of a tenth aspect is directed to the transcranial magnetic stimulation system of the first aspect, further provided with acquisition means that acquires position information representing position of the plurality of movable members at the current time, and wherein the receiving means receives at least one of position and attitude, constituting objectives for the coil section, as the desired change.

The invention of an eleventh aspect is directed to the transcranial magnetic stimulation system of the tenth aspect, wherein the guidance means performs guidance of the adjustment procedure using position information that has been acquired by the acquisition means.

The invention of a twelfth aspect is directed to the transcranial magnetic stimulation system of the eleventh aspect, wherein the guidance means determines whether or not position represented by the position information that has been acquired coincides with a position that will be guided using the adjustment procedure or coincides with being within a permissible range.

The invention of a thirteenth aspect is directed to the transcranial magnetic stimulation system of any one of the first to twelfth aspects, wherein the guidance means shows at least order in the plurality of adjustment mechanisms and adjustment direction of the adjustment mechanisms which should be adjusted.

The invention of a fourteenth aspect is directed to the transcranial magnetic stimulation system of any one of the first to thirteenth aspects, wherein the guidance means displays an image or a movie that emphasize or indicate the adjustment mechanisms or the movable members that are to be adjusted at the current point in time.

The invention of a fifteenth aspect is directed to the transcranial magnetic stimulation system of any one of the first to fourteenth aspects, wherein the guidance means provides guidance of the adjustment procedure using sound.

The invention of an sixteenth aspect is directed to the transcranial magnetic stimulation system of any one of the first to fifteenth aspects, wherein the mechanical section is controlled so that the coil section moves along a curved line corresponding to the surface of the head of a subject, as one movement direction.

The invention of a seventeenth aspect is directed to the transcranial magnetic stimulation system of any one of the first to sixteenth aspects, wherein the mechanical section is controlled so that the coil section moves in a vertical direction of a subject, as one movement direction.

The invention of an eighteenth aspect is directed to the transcranial magnetic stimulation system of any one of the first to seventeenth aspects, wherein the mechanical section is controlled so that the coil section moves along a direction towards substantially the center of the head of a subject, as one movement direction.

The invention of a nineteenth aspect is directed to a positioning assistance method for a mechanical section of a transcranial magnetic stimulation system, wherein
the mechanical section comprises a plurality of adjustment mechanisms that adjust position of a plurality of movable members, and determines position and attitude of the coil section by adjustment using the plurality of adjustment mechanisms, and wherein a device executes:
a receiving process of receiving desired change for at least one of position and attitude of the coil section, and
a guidance process of providing guidance of an adjustment procedure for at least one of the plurality of adjustment mechanisms so as to achieve the desired change that has been received.

The invention of a twentieth aspect is directed to a computer program for a positioning assistance method for a mechanical section of a transcranial magnetic stimulation system, wherein
the mechanical section comprises a plurality of adjustment mechanisms that adjust position of a plurality of movable members, and determines position and attitude of the coil section by adjustment using the plurality of adjustment mechanisms, and the computer program causes a computer to execute:
a receiving process of receiving desired change for at least one of position and attitude of the coil section, and
a guidance process of providing guidance of an adjustment procedure for at least one of the plurality of adjustment mechanisms so as to achieve the desired change that has been received. It should be noted that the computer program may be provided in a state of being stored in a computer readable storage medium, or may be provided in a state of being stored on a server and downloaded from the server.

The invention of a twenty-first aspect is directed to the transcranial magnetic stimulation system of the second aspect, further provided with detection means that detects contact state of the coil section with respect to the head, using a sensor section that includes one or more sensors, and wherein
the receiving means receives input of contact state that has been detected.

The invention of a twenty-second aspect is directed to the transcranial magnetic stimulation system of the twenty-first aspect, wherein
the detection means quantitatively detects the contact state, and
the guidance means obtains adjustment amount or target position for the adjustment mechanism based on contact state that has been quantitatively detected.

The invention of a twenty-third aspect is directed to the transcranial magnetic stimulation system of the twenty-first aspect or twenty-second aspect, wherein the contact state includes at least one of a one-side contact state where only part of an outer edge section of the coil section contacts the head, and an adhered state where the coil section contacts the head.

The invention of a twenty-fourth aspect is directed to the transcranial magnetic stimulation system of any one of the twenty-first aspect to twenty-fourth aspect, wherein the sensor section includes at least one among an imaging sensor, an ultrasonic sensor, an infrared sensor, a pressure sensor, a mechanical switch sensor, an electrostatic sensor, and an acceleration sensor.

### Effect of the Invention

According to the present invention, receiving means receives desired change for at least one of position and attitude of a coil section, and guidance means performs guidance of an adjustment procedure for at least one of a plurality of adjustment mechanisms that are included in a mechanical section that supports the coil section. As a result of this, an operator can adjust the coil section to a desired position and attitude simply by adjusting the adjustment mechanisms in accordance with the adjustment procedure that has been given as guidance, and it is possible to rapidly perform positioning of a mechanical section.

Also, according to the present invention an operator can master the adjustment technique for positioning the mechanical section early on by repeatedly receiving guidance of the adjustment procedure.

### Brief Description Of The Drawings

Fig. 1 is a drawing schematically showing the structure of a transcranial magnetic stimulation system of a first embodiment.
Fig. 2 is a drawing schematically showing the structure of a positioning mechanical section.
Fig. 3 is a drawing showing the structure of a physique adjustment mechanism and headgear.
Fig. 4 is a drawing showing adjustment direction of a coil section of headgear.
Fig. 5 is a drawing showing the structure of a control unit of the first embodiment.
Fig. 6 is a functional block diagram of the control unit of the first embodiment.
Fig. 7 is a drawing showing a one-side contact state for a parietal region side (upper side), as one example of a one-side contact state.
Fig. 8 is a flow chart showing flow of processing up to commencement of treatment, with the transcranial magnetic stimulation system of this embodiment.
Fig. 9 is a drawing showing flow of processing for performing guidance of an adjustment procedure.
Fig. 10 is a drawing showing one example of a one-side contact state selection screen.
Fig. 11 is one example of a flow chart showing an adjustment procedure.
Fig. 12 is a drawing showing a positional relationship between a head and a coil section in a case where a rotational axis of a roll direction is at a lower position than it should be.
Fig. 13 is a drawing showing a positional relationship between a head and a coil section in a case where a rotational axis of a roll direction is at the ideal position.
Fig. 14 is a drawing schematically showing the structure of a transcranial magnetic stimulation system of a second embodiment.
Fig. 15 is a drawing showing the structure of a control unit of the second embodiment.
Fig. 16 is a functional block diagram of the control unit of the second embodiment.
Fig. 17 is a flowchart showing flow of processing in a case where guidance is performed for an adjustment procedure using position information of respective adjustment mechanisms.
Fig. 18 is a display example 1 of a case where guidance is performed for an adjustment procedure in order to fix a parietal region side one-side contact state of the coil.
Fig. 19 is a display example 2 of a case where guidance is performed for an adjustment procedure in order to fix a parietal region side one-side contact state of the coil.
Fig. 20 is a display example 3 of a case where guidance is performed for an adjustment procedure in order to fix a parietal region side one-side contact state of the coil.
Fig. 21 is a display example 4 of a case where guidance is performed for an adjustment procedure in order to fix a parietal region side one-side contact state of the coil.
Fig. 22 is a flow chart showing flow of processing in a case where guidance is performed for an adjustment procedure in order to change the coil section to a target position and attitude.
Fig. 23 is a drawing schematically showing the structure of a transcranial magnetic stimulation system of a fourth embodiment.
Fig. 24 is a functional block diagram of the control unit of the fourth embodiment.

### Description of the Embodiments

Detailed description will now be given of embodiments of the present invention. It should be noted that the present invention is not limited to the embodiments described in the following. Also, in the embodiments below, elements having the same, equivalent, or common functions will be shown with the same reference numbers assigned.

### (First Embodiment)

A transcranial magnetic stimulation system of a first embodiment has a structure that is suitable for magnetic stimulation treatment, with the objective of pain relief for a subject, performs accurate positioning of a coil section, and is provided with a mechanical section that replicate that positioning.

Also, the transcranial magnetic stimulation system of the first embodiment is configured so that if a desired change for position and attitude of the coil section is input, guidance is performed for an adjustment procedure so as to achieve that change for assisting with rapid positioning of the mechanical section.

The structure of the transcranial magnetic stimulation system of the first embodiment will be described.

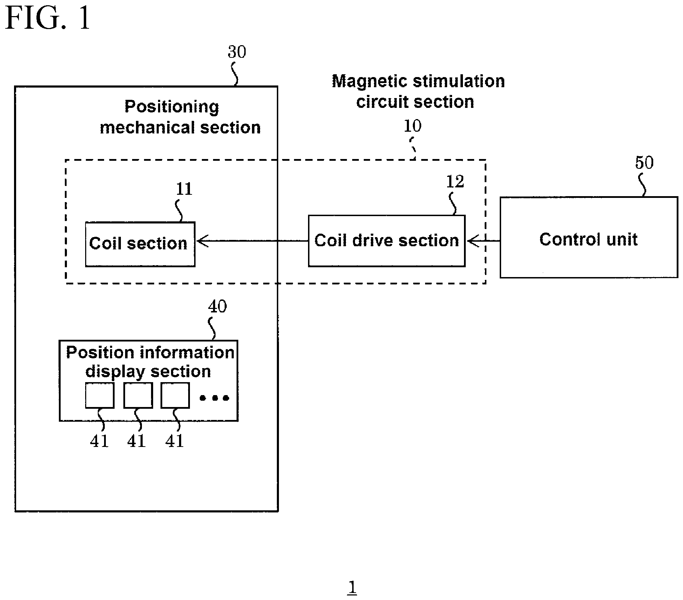

Fig. 1 is a drawing schematically showing the structure of a transcranial magnetic stimulation system 1 of a first embodiment.

As shown in Fig. 1, the transcranial magnetic stimulation system 1 comprises a magnetic stimulation circuit section 10, a positioning mechanical section 30, a position information display section 40, and a control unit 50.

The magnetic stimulation circuit section 10 is configured so as to apply stimulation due to induced current forming a variable magnetic field (in the following specification this will be referred to simply as "magnetic stimulation") to the brain of a subject. The magnetic stimulation circuit section 10 comprises a coil section 11, and a coil drive section 12 that is connected to the coil section 11. The coil section 11 has a head contacting surface, and this surface is arranged so as to be close to or in contact with a portion, where is it desired to apply magnetic stimulation, on the head (brain) of a subject. The coil section 11 forms a magnetic field if electrical current flows. The coil drive section 12 causes a pulse shaped electrical current to flow in the coil section 11 to form a variable magnetic field momentarily at a location where there is a given positional relationship with the coil section 11. An induced current due to this variable magnetic field constitutes magnetic stimulation to the brain of a subject.

The positioning mechanical section 30 is a mechanical section for adjusting positional relationships between the brain of the subject and respective parts within the system (particularly the coil section 11), when applying magnetic stimulation to the subject, as required. It should be noted that while, in Fig. 1, the coil section 11 and the position information display section 40 are included within the block representing the positioning mechanical section 30, this is because Fig. 1 is for showing an example where the coil section 11 and the position information display section 40 are physically close to the positioning mechanical section 30, and is not for defining an inclusion relation between these elements.

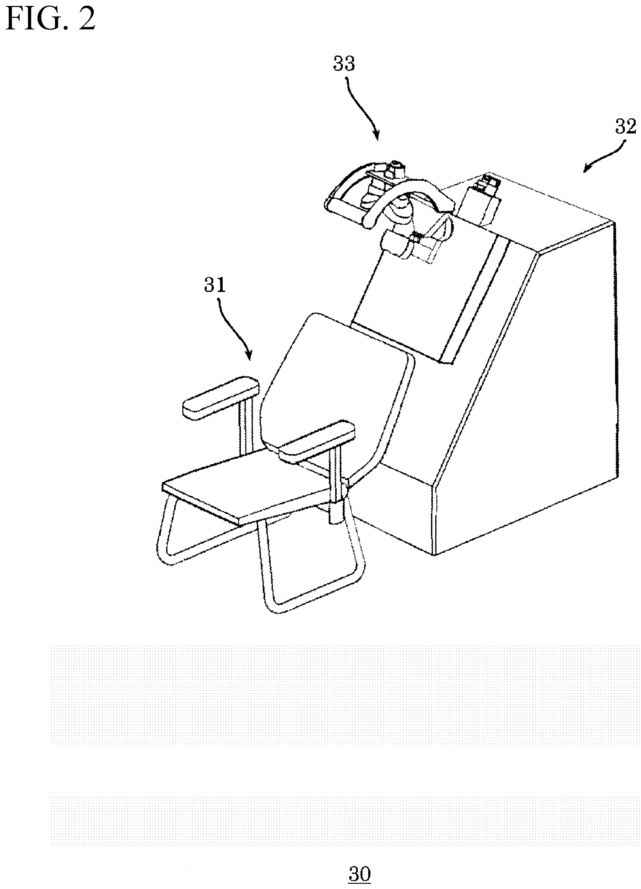

The structure of the positioning mechanical section 30 is schematically shown in Fig. 2.

As shown in Fig. 2, the positioning mechanical section 30 comprises a chair 31, a physique adjustment mechanism 32, and headgear 33.

The chair 31 has a seat surface and a back, and is configured so that it is possible keep the entire body of a subject in a seated attitude.

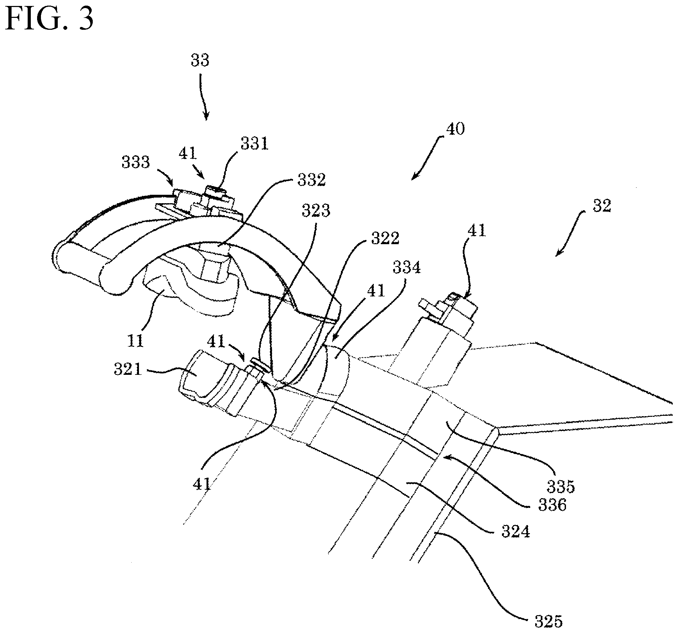

The structure of the physique adjustment mechanism 32 and the headgear 33 is shown in Fig. 3.

The physique adjustment mechanism 32 is configured so that it is possible to support the occipital region of a subject who is seated on the chair 31, in accordance with physique of the subject.

As shown in Fig. 3, the physique adjustment mechanism 32 comprises an occipital region rest 321, a forward and backward direction adjustment mechanism 322, a vertical direction adjustment mechanism 323, a physique adjustment guide 324, and a height adjustment mechanism 325.

The occipital region rest 321 is configured so that it is possible to support the subject's occipital region, and has a concave curved surface that receives the occipital region. The occipital region rest 321 is supported by the forward and backward direction adjustment mechanism 322 and the vertical direction adjustment mechanism 323. The forward and backward direction adjustment mechanism 322 can adjust position of the occipital region rest 321 in the forward and backward direction of the subject. The vertical direction adjustment mechanism 323 can adjust the position of the occipital region rest 321 in the vertical direction of the subject who is seated on the chair 31 (direction of the back of the chair 31). The forward and backward direction adjustment mechanism 322 and the vertical direction adjustment mechanism 323 are supported by the physique adjustment guide 324.

The physique adjustment guide 324 is supported by the height adjustment mechanism 325. The height adjustment mechanism 325 can adjust the height of the physique adjustment guide 324 in the vertical direction of a subject who is seated on the chair 31.

The headgear 33 supports the coil section 11, and is configured so that it is possible to adjust position and attitude of the coil section 11.

As shown in Fig. 3, the headgear 33 comprises a pushing direction adjustment mechanism 331, a yaw direction adjustment mechanism 332, a pitch direction adjustment mechanism 333, a roll direction adjustment mechanism 334, a headgear guide 335 and a attaching/detaching mechanism 336.

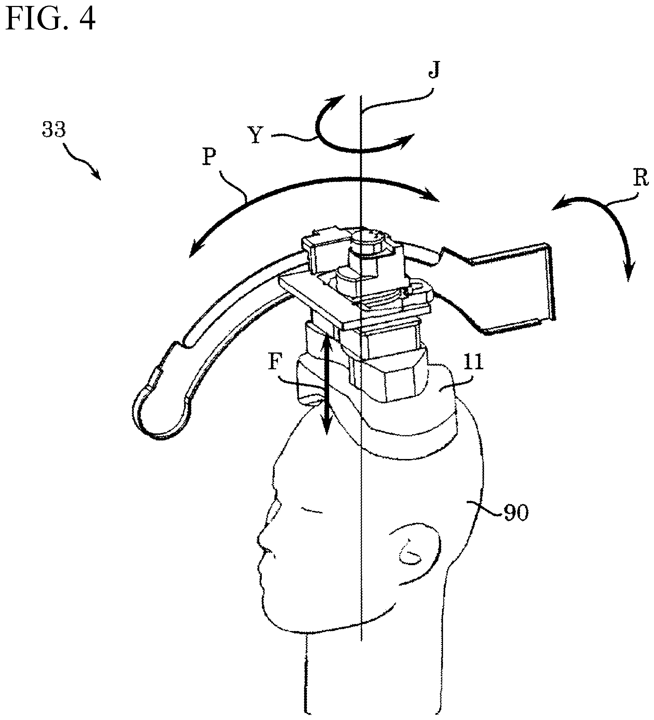

Adjustment directions of the coil section 11 in the headgear 33 are shown in Fig. 4. With this example, as shown in Fig. 4, there are four adjustment directions of the coil section 11, namely the pushing direction F, yaw direction Y, pitch direction P, and roll direction R. The pushing direction F is a direction in which the coil section 11 is pushed against the head of the subject 90, namely direction J towards substantially the center of the head of the subject 90. The yaw direction Y is a rotation direction with a direction towards substantially the center of the head of the subject 90 as an axis. The pitch direction P is a curving direction in accordance with the surface of the head of the subject 90 in the forward and backward direction. The roll direction R is a curving direction in accordance with the surface of the head of the subject 90 in the lateral direction. It should be noted that a curving direction in accordance with the surface of the head is a curving direction, in searching for an optimum stimulation position, such that it is possible to cause the position of the coil section 11 to move smoothly along the surface of the head. For example, the curving direction is a direction of curving having a curvature that approximates to the curvature of the surface of a head model having an average head size and shape for an adult, or a curvature that is slightly smaller (gentler) than that.

As shown in Fig. 3, the coil section 11 is supported by the pushing direction adjustment mechanism 331. The pushing direction adjustment mechanism 331 can adjust position of the coil section 11 in the pushing direction F. The pushing direction adjustment mechanism 331 is supported by the yaw direction adjustment mechanism 332. The yaw direction adjustment mechanism 332 can adjust rotation position of the pushing direction adjustment mechanism 331 in the yaw direction Y. The yaw direction adjustment mechanism 332 is supported by the pitch direction adjustment mechanism 333. The pitch direction adjustment mechanism 333 can adjust position of the yaw direction adjustment mechanism 332 in the pitch direction P. The pitch direction adjustment mechanism 333 is supported by the roll direction adjustment mechanism 334. The roll direction adjustment mechanism 334 can adjust inclination angle of the pitch direction adjustment mechanism 333 in the roll direction R. The roll direction adjustment mechanism 334 is supported by the headgear guide 335. The headgear guide 335 is supported by the attaching/detaching mechanism 336. The attaching/detaching mechanism 336 can cause the headgear guide 335 to slide in a detachable direction, with respect to the physique adjustment guide 321, in the vertical direction of the subject 90 seated on the chair 31.

As shown in Fig. 3, the position information display section 40 displays position information of movable members of each of the adjustment mechanisms constituting the positioning mechanical section 30. The position information display section 40 has a plurality of position display sections 41. Position display sections 41 are provided on a one-to-one basis for a single adjustment mechanism, namely a single movable member. A position display section 41 displays information representing position of a corresponding movable member in a form that can be visually confirmed. An operator can grasp that position by visually confirming the position information that this position display section displays.

It is possible to consider digital or analog indicators, for example, as the position display sections 41. As indicators, it is possible to consider, for example, a scale, an angle meter or a position indicator. A position indicator is for displaying movement amount (feed amount) of a movable member from a reference position with a counter or liquid crystal screen.

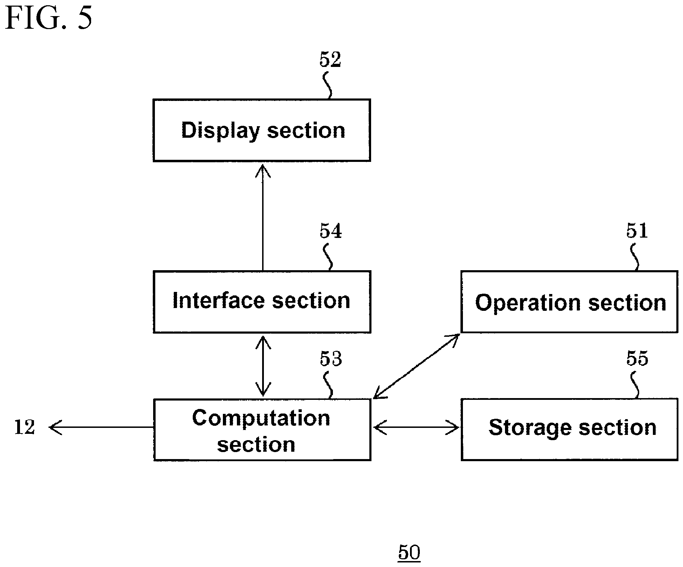

A hardware structure example of a control unit of the first embodiment is shown in Fig. 5.

As shown in Fig. 5, the control unit 50 is constituted by a computer, for example. The control unit 50 has, as main sections, an operation section 51, a display section 52, a computation section 53, an interface section 54, and a storage section 55.

The operation section 51 receives operations from an operator to perform various inputs. As the operation section 51 it is possible to consider, for example, a keyboard or a mouse etc.

The display section 52 displays various information on a screen. As the display section 52 it is possible to consider, for example, a liquid crystal display or an organic EL display etc. It should be noted that the operation section 51 and the display section 52 may be made into a touch panel type display in which these two sections are integrally constructed. The control unit 50 may also be implemented using a mobile terminal in which such a touch panel type display is provided. In this case, since the operator will be holding the mobile terminal at a given location, it is possible to perform adjustment of the positioning mechanical section 30 etc. while performing operation and perusal of the screen.

The computation section 53 is a processor, and is a semiconductor IC known as an MPU or CPU. The computation section 53 may have cache memory.

The interface section 54 performs exchange of various data between the operation section 51 and the display section 52, and the computation section 53.

The storage section 55 stores various data, and stores programs etc. for causing execution in the computation section 53. As the storage section 55 it is possible to consider, for example, a HDD (Hard Disc Drive) or on SSD (Solid State Device) that uses flash memory.

The control unit 50 functions as a plurality of functional blocks, by causing the computation section 53 to read out and execute given programs that are stored in the storage section 55.

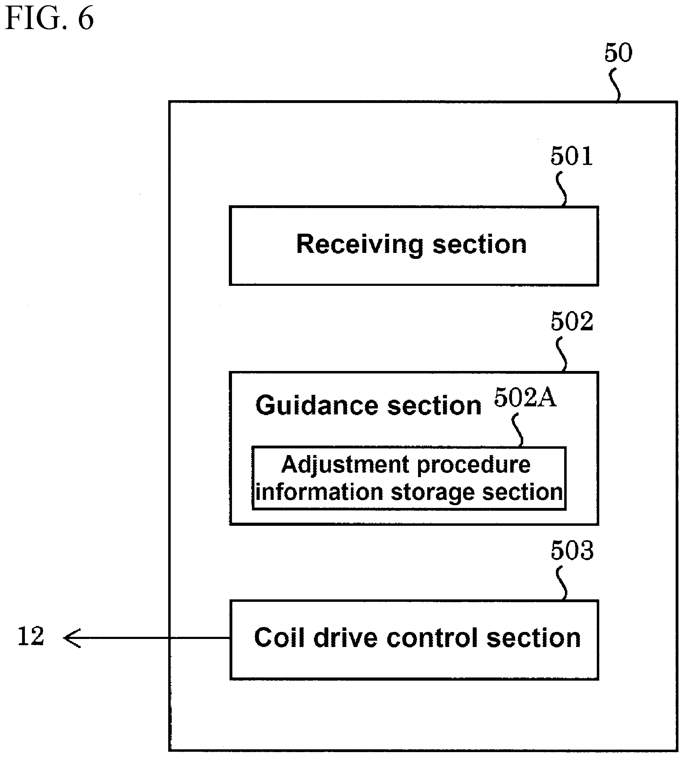

A functional block diagram of the control unit of the first embodiment is shown in Fig. 6.

Here, as shown in Fig. 6, as functional blocks the control unit 50 has a receiving section 501, guidance section 502, and a coil drive control section 503.

The receiving section 501 receives various input as a result of operation by the operator. With this example, the receiving section 501 receives input such as magnetic stimulation conditions, magnetic stimulation commencement instruction, treatment commencement instruction etc. The magnetic stimulation conditions include magnetic stimulation strength and a generation pattern for magnetic stimulation at the time of performing treatment etc.

Also, with this example, the receiving section 501 receives input of desired change in at least one of position and attitude of the coil section 11. This change input is performed by the operator selecting one from among a plurality of types of change previously registered.

The above-mentioned "change" includes change for improving or resolving a given contact state of the coil section 11 with the subject's head.

With this example, "change" is change for resolving a one-side contact state of the coil section 11. The one-side contact state is a state such that only a part of an outer edge section of a head contact surface of the coil section 11 contacts the head surface, because the head contact surface of the coil section 11 is inclined with respect to the head surface of the subject.

Also, with this example "a plurality of types of change" is change, for each of a plurality of types of one-side contact state, for resolving that one-side contact state. A plurality of types of one-side contact state are classified in accordance with what part of the outer edge section of the head contact surface of the coil section 11 is in contact with the subject's head. With this example, as locations where contact the coil section 11 contacts the subject's head, six types are considered, namely at the parietal region (upper), at the temporal region (lower), at the forehead (front), at the occipital region (rear), between the temporal region and the forehead (inclined downward to the front), and between the parietal region and the occipital region (inclined upward to the rear). It should be noted that these types of location for contact are one example, and the present invention is not limited to these.

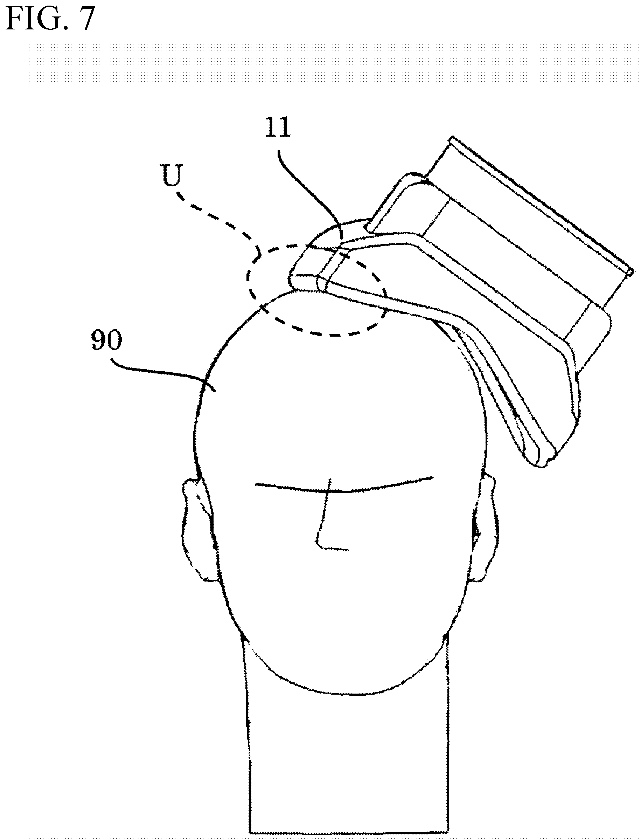

A one-side contact state for a parietal region (upper side), as one example of a one-side contact state, is shown in Fig. 7. In the case of pain relief treatment, there is a stimulation region at a motor area that exists close to a central part of the brain in a forward and backward direction. As a one-side contact state for the parietal region, it is conceivable that, as shown in Fig. 7, a parietal region side (upper side) U of the outer edge section of the head contact surface of the coil section 11 will often contact a region between the parietal region and the temporal region of the subject 90.

If a desired change amount for at least one of position and attitude of the coil section 11 is received by the receiving section 501, the guidance section 502 provides guidance for an adjustment procedure so as to achieve that change. As shown in Fig. 6, the guidance section 502 has an adjustment procedure storage section 502A that stores a plurality of types of adjustment procedure information. "a plurality of types of adjustment procedure information" are respectively associated with each of a "plurality of types of change". The guidance section 502 reads out adjustment procedure information corresponding to desired change that has been input, from the adjustment procedure storage section 502A, and performs visual or aural guidance for the adjustment procedure represented by that adjustment procedure information. In the case of providing visual guidance of the adjustment procedure, drawings and tables showing the adjustment procedure may be simply displayed on the display section 52, as hardware, or navigation that makes the procedure easy to understand may be performed using a movie. Alternatively, a light emitting part may be provided in each adjustment mechanism, and control performed so that light emitting parts of adjustment mechanisms that are required in adjustment are sequentially lit up.

It should be noted that specified computation may be performed based on desired change that has been input, and the guidance section 502 may obtain and perform guidance for an adjustment procedure so as to achieve the desired change based on the result of that computation.

The coil drive control section 503 controls the coil drive section 12 in response to various commands that have been received by the receiving section 501. With this example, the coil drive control section 503 controls the coil drive section 12 to apply one-shot magnetic stimulation to the subject 90 in accordance with magnetic stimulation conditions that have been set, in response to a one-shot magnetic stimulation command. The coil drive control section 503 may also control the coil drive section 12 to apply a plurality of bursts of magnetic stimulation in a specified pattern to the subject 90 in accordance with magnetic stimulation conditions that have been set, in response to a treatment commencement instruction.

It should be noted that the receiving section 501 and the guidance section 502 of this embodiment are respectively one example of receiving means and guidance means of the present invention.

Processing flow for the transcranial magnetic stimulation system 1 of this embodiment will now be described.

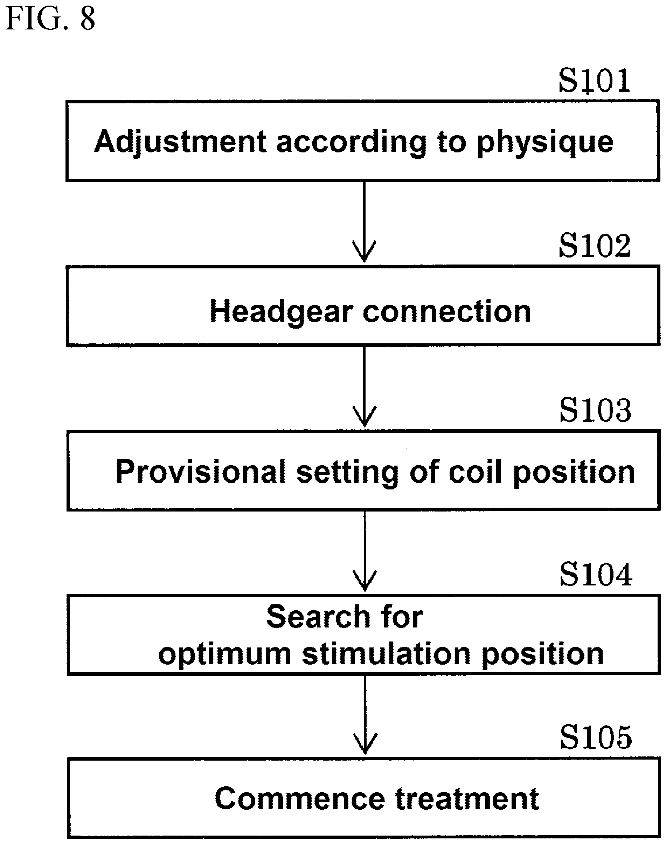

Fig. 8 is a flow chart showing flow of processing up to commencement of treatment, with the transcranial magnetic stimulation system 1 of this embodiment.

In step S101, physique adjustment is performed. Specifically, the operator first has the subject 90 sit on the chair 31. At this time, if the headgear 33 is connected to the physique adjustment guide 324, the attaching/detaching mechanism 336 is operated and the headgear 33 is raised upwards. Next, the operator adjusts the height of the physique adjustment guide 324 to conform to the physique of the subject 90, using the height adjustment mechanism 325. The operator also adjusts position of the occipital region rest 321 forward and backward, and up and down, so as to line up with the occipital region of the subject 90, using the forward and backward direction adjustment mechanism 322 and the vertical direction adjustment mechanism 323.

In step S102, connection of the headgear 33 is performed. Specifically, the operator operates the attaching/detaching mechanism 336 to lower the headgear guide 335 downwards, and connect to the physique adjustment guide 321.

In step S103, provisional setting of coil position is performed. Specifically, the operator provisionally sets position of the coil section 11 by adjusting each adjustment mechanism of the headgear 33. A position that is provisionally set is a position corresponding to a stimulation region that has been designated according to pain area with reference to a medical image of the head of the subject 90. It should be noted that, in this provisional setting of coil position, adjustment of each adjustment mechanism as mentioned above may be unsuccessful, and further a situation arises whereby an efficient adjustment procedure is difficult to comprehend. Dealing with this case will be described later.

In step S104, search for optimum stimulation position is performed. Specifically, the operator performs input operations with the receiving section 501 (operation section 51 in the hardware), and sets the magnetic stimulation strength comparatively strong. Then, while adjusting each adjustment mechanism of the head gear 33 to vary position of the coil section 11 a little bit at a time in the vicinity of the provisionally set position, a magnetic stimulation commencement instruction is input each time in the receiving section 501. The coil drive control section 503 successively responds to the magnetic stimulation commencement instructions to generate magnetic stimulation. The operator looks at a twitch response of the subject 90 each time magnetic stimulation is generated. A position at which the strongest twitch response appears is then set as the optimum stimulation position.

In step S105, treatment is commenced. Specifically, the operator performs an input operation with the receiving section 501 (operation section 51), and returns magnetic stimulation strength to the optimum strength for treatment. A treatment commencement instruction is then input at the receiving section 501. The coil drive control section 503 controls the coil drive section 12, and magnetic stimulation for treatment in accordance with magnetic stimulation conditions that have been set is commenced.

Here, the provisional setting of coil position in step S103 will be described for handling a case where adjustment has been unsuccessful.

With provisional setting of coil position, attitude of the coil section 11 is often adjusted so as to adhere a head contact surface of the coil section 11 to the subject's head, so as to convey magnetic stimulation from the coil section 11 to the stimulation region efficiently over the shortest distance. Also, while strength of a variable magnetic field from the coil section 11 (that is, strength of induced current) is also dependent on coil shape, it is generally known to have directivity, and direction of the magnetic stimulation is accurately controlled by adhering the coil section 11 to the subject's head.

However, in actual fact there are cases where provisional setting of coil position is not successful, and the coil section 11 is in a one-side contact state with the subject's head. This can be considered to be due to the fact that it is difficult to intuitively grasp movement of the coil section 11 because a positional relationship between the coil section 11 and the head surface of the subject is not decided only by adjustment of each adjustment mechanism of the head gear 33 and because adjustment direction of each adjustment mechanism is not simple due to various circumstances and restrictions etc. In particular, if a curving direction corresponding to the head surface of the subject is included in one adjustment direction, degree of difficulty of adjustment is increased. There are efficient adjustment procedures for fixing the one-side contact state of a coil that are almost constant according to a one-side contact state pattern, but generally it is not that easy for the operator to manually attain this adjustment procedure instantly. Also, a surface of the subject's head and a treatment location within the brain are generally apart to a certain extent. If the direction of magnetic stimulation deviates from the correct direction because the coil section 11 is not adhered to the subject's head (for example, a one-side contact state arises), then it is also possible to consider that a desired electrical current stimulation will not be generated at the treatment location due to being apart, as described previously. Accordingly, in order to increase treatment efficiency it is desirable to fix this type of one-side contact state, and accurately set a positional relationship between the coil section 11 and the subject's head.

Therefore, if this type of one-side contact state of the coil arises, the operator utilizes the function of providing guidance for the adjustment procedure in order to fix this one-side contact state, by operating the receiving section 501 (operation section 51) of the control unit 50.

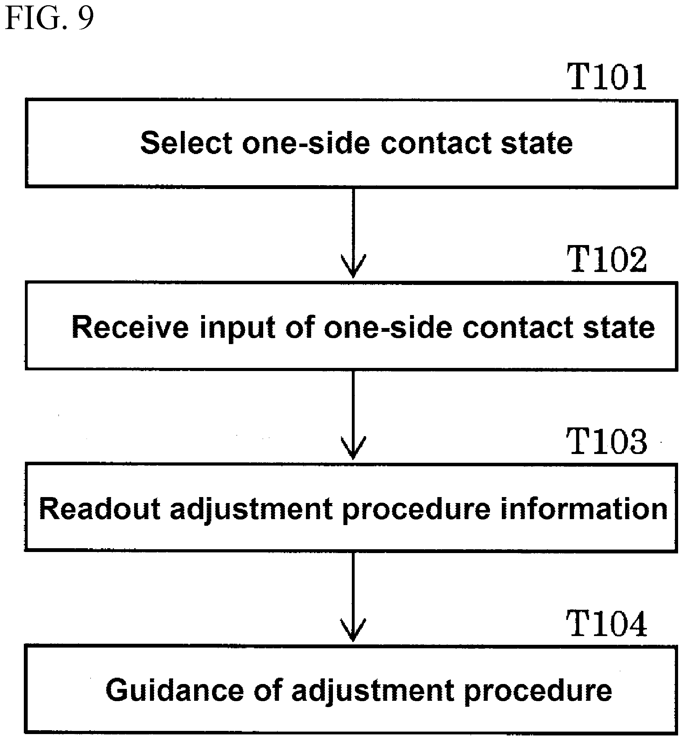

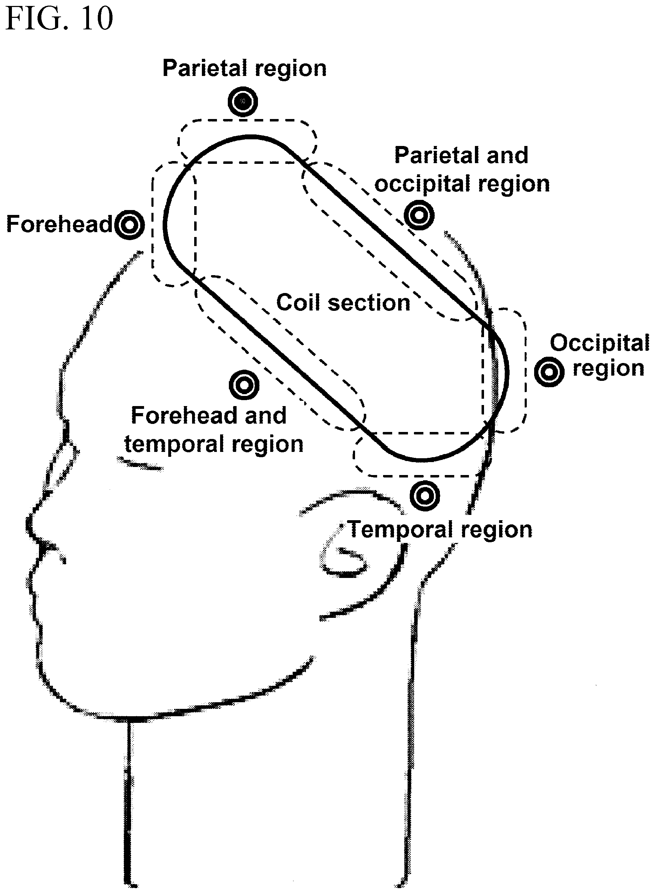

Flow of processing for providing guidance of the adjustment procedure is shown in Fig. 9. Also, one example of a one-side contact state selection screen is shown in Fig. 10.

First, on a one-side contact state selection screen such as shown in Fig. 10 that is displayed on the receiving section 501 (display section 52), the operator selects a one-side contact state at the current time, from among a plurality of alternatives, by operating the receiving section 501 (operation section 51) (step T101). In this way, the receiving section 501 receives a one-side contact state that has been selected, as "input of one-side contact state it is desired to fix" (step T102). Once this is done, the guidance section 502 reads out adjustment procedure information corresponding to the one-side contact state that has been selected, from the adjustment procedure storage section 502A (step T103). The guidance section 502 then performs aural or visual guidance of the adjustment procedure represented by that adjustment procedure information (step T104).

The guidance section 502 displays the adjustment procedure, for example, using the display section 52, as hardware. As adjustment procedure display, it is possible to consider displaying a flow chart that shows which movable members of which adjustment mechanism are adjusted, and in what order and in which direction they are adjusted. It should be noted, regarding adjustment procedure guidance, that by how much adjustment is performed may be guided in a case where guidance is possible, or may be left to the operator's discretion.

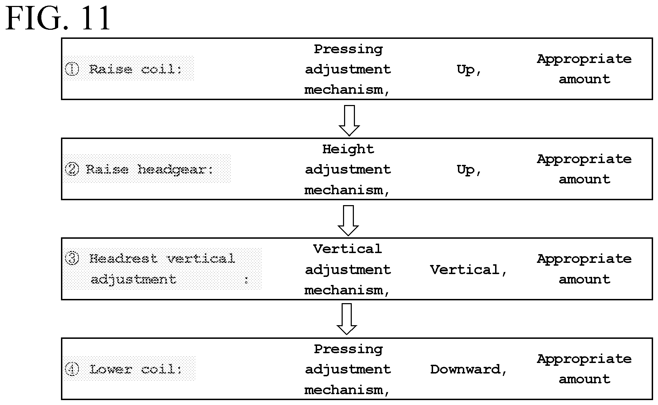

One example of a flowchart for display using the guidance section 502 in order to show an adjustment procedure is shown in Fig. 11. The flowchart shown in Fig. 11 is for showing an adjustment procedure in order to fix a one-side contact state at the "parietal region". It should be noted that in Fig. 11 "appropriate amount" means an amount considered appropriate by the operator. Instead of "appropriate amount", a specific movement amount for a movable member of an adjustment mechanism may use a scale numerical value. This adjustment procedure is as follows.

First, in step 1 adjustment is performed to move the coil section 11 upward (in a direction away from the head) using the pushing direction adjustment mechanism 331. This is so as to move the coil section 11 away from the head, so that the coil section 11 will fail to contact the head even if it is moved.

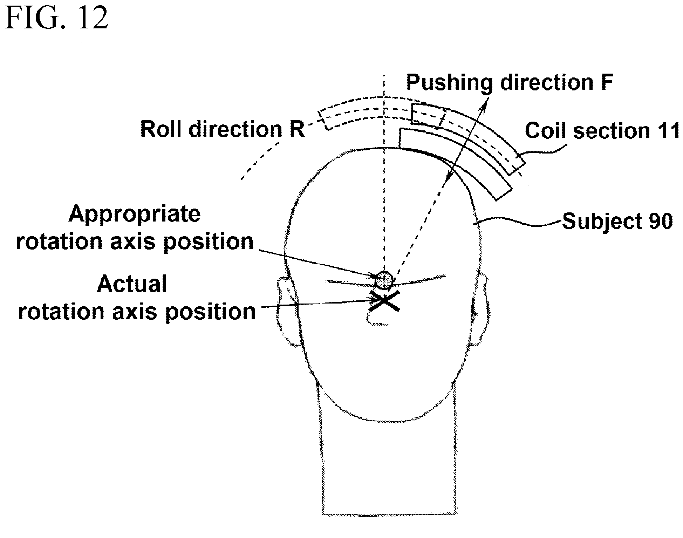

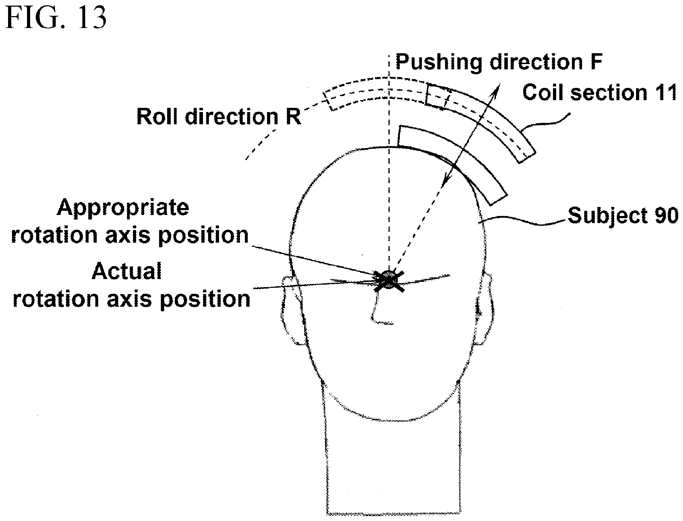

Next, in step 2 adjustment to move the height of the physique adjustment guide 324 upward is performed using the height adjustment mechanism 325. A case where the parietal region side of the coil section 11 gets in a one-side contact state is caused by position of a rotational axis for the roll direction R, of the coil section 11, being at a position that is lower than a proper position that fits the size or shape of the head of the subject 90, as shown in Fig. 12. A locus of rotation of the coil section 11 in this case moves further apart from the head surface toward the temporal region side, which means that even if position in the pushing direction F is adjusted in this state there will be one-side contact. Therefore, here, as shown in Fig. 13, the physique adjustment guide 324 is moved upwards so as to raise the position of the coil section 11 on the rotational axis for roll direction R and returned to the correct position. A locus of rotation of the coil section 11 in this case is substantially parallel to the head surface, and so one-side contact does not arise even if position is adjusted in the pushing direction F in this state.

Next, in step 3 vertical direction adjustment is performed so that the occipital region rest 321 lines up with the occipital region of the subject 90 without any feeling of discomfort, using the vertical direction adjustment mechanism 323. This is because it is necessary to perform adjustment of the occipital region rest 321 again because height of the occipital region rest 321 has also been changed as a result of changing the height of the physique adjustment guide 324 in step 2.

First, in step 4 adjustment is performed to move the coil section 11 downward (in a direction approaching the head) using the pushing direction adjustment mechanism 331. This is to return to the original height because the coil section 11 was separated from the subject's head in step 1.

**Table 1**

| # | mechanism | Adjustment location | One side contact location | | | | | | | | |
|---|---|---|---|---|---|---|---|---|---|---|---|
| | | | parietal region | | | temporal region | | | occipital region | | |
| | | | step | direction | Movement amount | step | direction | Movement amount | step | direction | Movement amount |
| 1 | Body type adjustment mechanism 32 | Occipital rest forward and backward direction 322 | - | - | - | - | - | - | 2 | forward | 2.6 |
| 2 | | Occipital rest vertical direction 323 | 3 | down | 2.0 | 3 | up | 8.0 | - | - | - |
| 3 | | height 325 | 2 | up | 2.1 | 2 | down | 8.2 | - | - | - |
| 4 | Headgear 33 | Pressing direction 325 | 1 | up | 10.0 | 1 | up | 10.0 | 1 | up | 10.0 |
| | | | 4 | down | 8.5 | 5 | down | 4.3 | 4 | down | 9.5 |
| 5 | | yaw direction 332 | - | - | - | - | - | - | - | - | - |
| 6 | | pitch direction 333 | - | - | - | - | - | - | 3 | forward | 4.0 |
| 7 | | roll direction 334 | - | - | - | 4 | left | 1.9 | - | - | - |

One example of results of having adjusted one-side contact at the parietal region that has been shown up to now, and results of having adjusted one-side contact at other locations (here the temporal region and the occipital region), are shown in table 1. It should be noted that "step" within the table means operational procedure. Accordingly, the fact that there is completion at step 4 means that adjustment is completed with four procedures. Also, "movement amount" within the table is represented by unit quantity of movement amount in this system, with yaw direction 332, pitch direction 333, and roll direction 334 being curved movement amounts (units: °) along each adjustment mechanism, and the others being linear movement amounts (units: mm)

In this way it is possible for the operator to fix the one-side contact state of the coil by simply performing adjustments in accordance with an adjustment procedure that has been displayed.

According to the first embodiment above, the receiving section 501 receives desired change with regard to position and attitude of the coil section 11, and the guidance section 502 provides guidance for an adjustment procedure so as to achieve that change, which means that the operator can achieve a desired change in position and attitude of the coil section 11 simply by performing adjustments in accordance with the adjustment procedure for which guidance has been provided, and the operator can rapidly perform positioning of the positioning mechanical section 30.

In particular, with the first embodiment, a plurality of types of adjustment mechanism include adjustment mechanisms for moving movable members along a curve direction corresponding to the surface of a subject's head. With this embodiment, with the adjustment mechanisms in their entirety (namely with the positioning mechanical section 30), it is possible to adjust position and attitude of the coil section 11 with at least four degrees of freedom, namely for yaw, pitch, roll, and translation (pushing direction). Regarding position of the coil section 11 in this case, an adjustment procedure that involves not only movement along a coordinate axis of an orthogonal system, which is normal and familiar, but that is also for performing movement in a desired direction and change in orientation to a desired rotation direction, is difficult to imagine intuitively. Therefore, with this first embodiment, the contribution to speeding up adjustment as a result of having guidance for the adjustment procedure is significant.

Also, according to the first embodiment, an operator can ascertain the structure of the positioning mechanical section 30 early on by repeatedly receiving guidance of adjustment procedures. Further, it is possible to learn adjustment techniques for positioning the positioning mechanical section 30 early on.

### (Second Embodiment)

A transcranial magnetic stimulation system of the second embodiment is configured to convert position information of movable members of each adjustment mechanism of a positioning mechanical section to signals and transmit the signals to a control unit, and to perform guidance of an adjustment procedure for the positioning mechanical section using the position information.

The structure of the transcranial magnetic stimulation system of the second embodiment will be described.

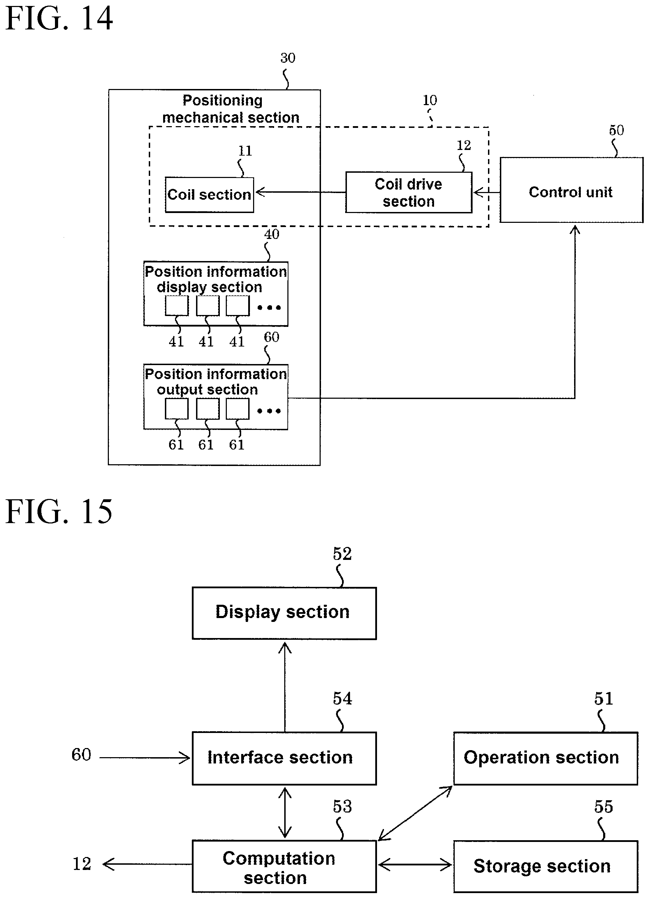

Fig. 14 is a drawing schematically showing the structure of a transcranial magnetic stimulation system 2 of the second embodiment.

As shown in Fig. 14, the transcranial magnetic stimulation system 2 comprises a magnetic stimulation circuit section 10, a positioning mechanical section 30, a position information display section 40, a position information output section 60, and a control unit 50.

The magnetic stimulation circuit section 10, positioning mechanical section 30, and position information display section 40 have the same structure as for the first embodiment. Description of the structure of these sections is therefore omitted here.

The position information output section 60 outputs position information of movable members of each adjustment mechanism of the positioning mechanical section 30 as signals. The position information output section 60 is connected to the control unit 50, and the signals are transmitted to the control unit 50. Connection between the position information output section 60 and the control unit 50 may be wired or wireless.

The position information output section 60 has a plurality of position detection sections 61. Position detection sections 61 are provided on the basis of at least one for one adjustment mechanism. A position detection section 61 detects position of a movable member of a corresponding adjustment mechanism and outputs information representing that position as a signal using light or electricity etc.

As a position detection section 61, it is possible to consider, for example, an inclination sensor, rotary encoder, wire type encoder, optical or magnetic linear encoder, or a position indicator having an output port, etc.

A hardware structure example of a control unit of the second embodiment is shown in Fig. 15.

As shown in Fig. 15, similarly to the first embodiment, the control unit 50 has, as main sections, an operation section 51, a display section 52, a computation section 53, an interface section 54, and a storage section 55.

The interface section 54 receives signals that have been transmitted from the position information output section 60, and sends these signals to the computation section 53.

The computation section 53 processes position information that has been sent from the interface section 54. Each hardware element of the devices of the second embodiment can be constructed in the same way as the previously described first embodiment apart from the above described points.

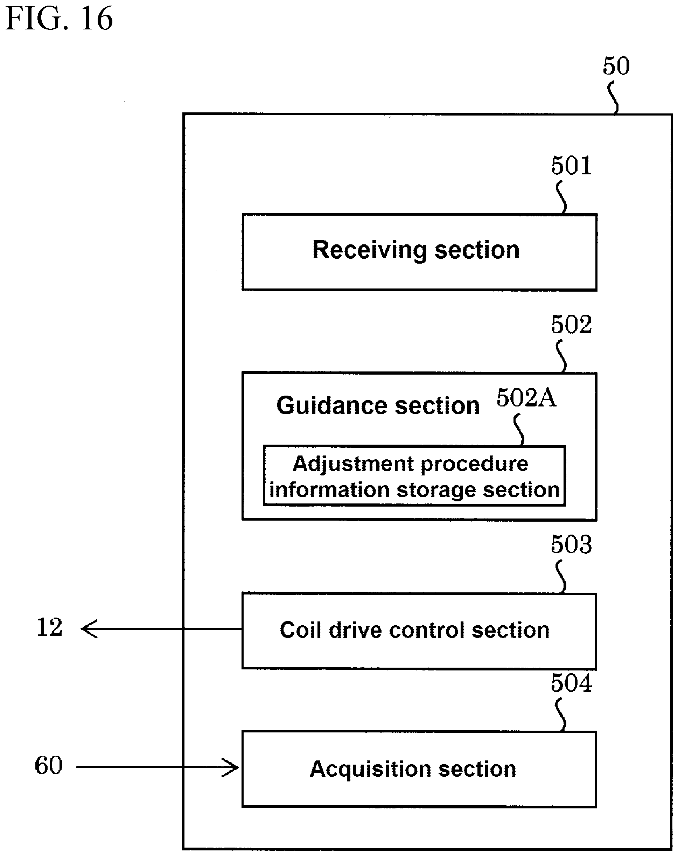

A functional block diagram of the control unit of the second embodiment is shown in Fig. 16.

Here, as shown in Fig. 16, as functional blocks the control unit 50 has a receiving section 501, guidance section 502, coil drive control section 503 and an acquisition section 504.

The receiving section 501 receives input such as magnetic stimulation conditions, magnetic stimulation commencement instruction, treatment commencement instruction etc. as a result of operation by the operator. Also, as a result of the operator selecting one from among a plurality of alternatives that represent one-side contact states of the coil section 11, the receiving section 501 receives input of that one-side contact state.

The acquisition section 504 acquires position information of movable members of each adjustment mechanism that has been sent from the position information output section 60 by means of signals, as current position information. Acquisition of the current position information is repeatedly performed in comparatively short cycles, and successively updated.

If a one-side contact state of the coil section 11 is input, the guidance section 502 performs guidance for an adjustment procedure for resolving that one-side contact state using position information of movable members for each adjustment mechanism that has been acquired by the acquisition section 504. Specifically, together with displaying the adjustment procedure, current position of the movable members of each adjustment mechanism is recognized, adjustment progress is monitored, and appropriate guidance is performed at each point in time.

It should be noted that the receiving section 501, guidance section 502 and acquisition section 504 of this embodiment are respectively one example of receiving means, guidance means and acquisition means of the present invention.

In the following, description will be given of processing flow in a case where guidance is provided for an adjustment procedure for provisional setting of coil position, using position information of each adjustment mechanism.

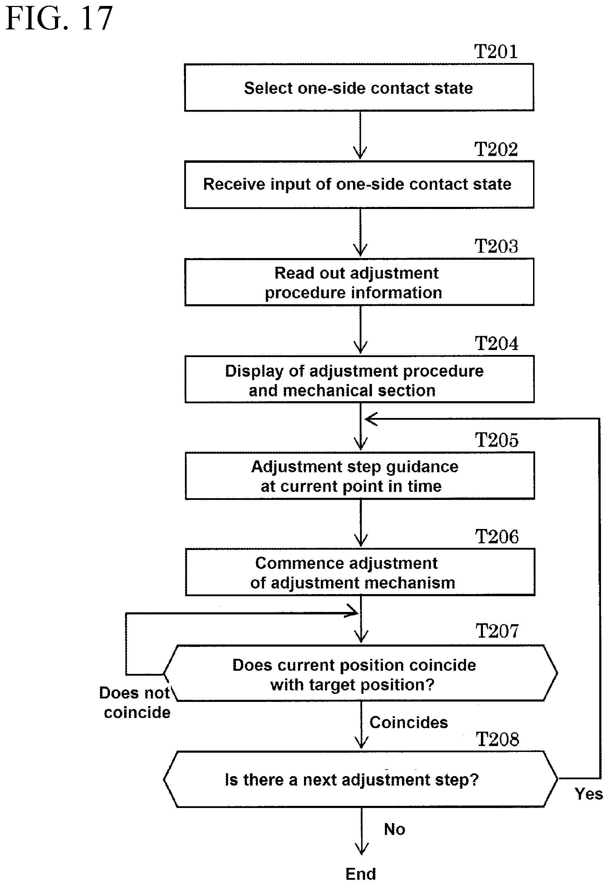

Fig. 17 is a flowchart showing flow of processing in a case where guidance is performed for an adjustment procedure using position information of each adjustment mechanism.

In step T201 a one-side contact state is selected. Specifically, the operator selects a current one-side contact state from among a plurality of alternatives.

In step T202, input of one-side contact state is received. Specifically, the receiving section 501 receives input of the one-side contact state that has been selected.

In step T203 adjustment procedure information is read out. Specifically, the guidance section 502 reads out adjustment procedure information representing an adjustment procedure for resolving the one-side contact state that was selected from the adjustment procedure storage section 502A.

In step T204 the adjustment procedure and a conceptual diagram of the mechanical section are displayed. Specifically, the guidance section 502 displays a flowchart showing the adjustment procedure based on the adjustment procedure information that has been read out. For example, which adjustment mechanisms are adjusted, and the order and direction in which they are adjusted, are divided into respective adjustment steps for each adjustment mechanism, and displayed. The guidance section 502 also displays a conceptual diagram in which each adjustment mechanism of the positioning mechanical section 30 has been modeled.

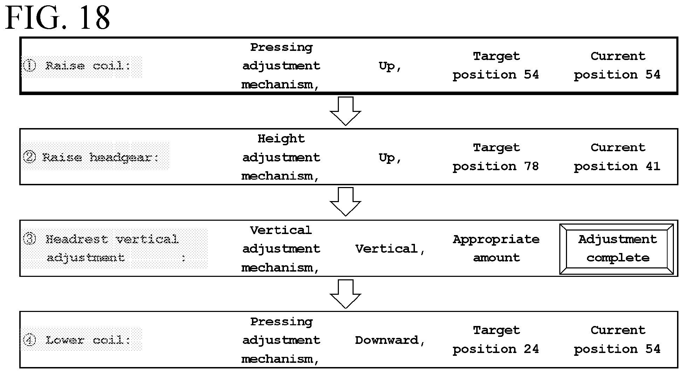

In step T205, guidance is provided for an adjustment step at the current point in time. Specifically guidance section 502 displays an adjustment step for an adjustment mechanism that is the subject of adjustment at the current point in time, emphasized within the flowchart that shows the adjustment procedure, as shown in Fig. 18. It should be noted that in the flowchart of Fig. 18, numerical values for target position and current position represent values of a scale that corresponds to position of movable members of the adjustment mechanism. "appropriate amount" means an amount that the operator considers to be appropriate.

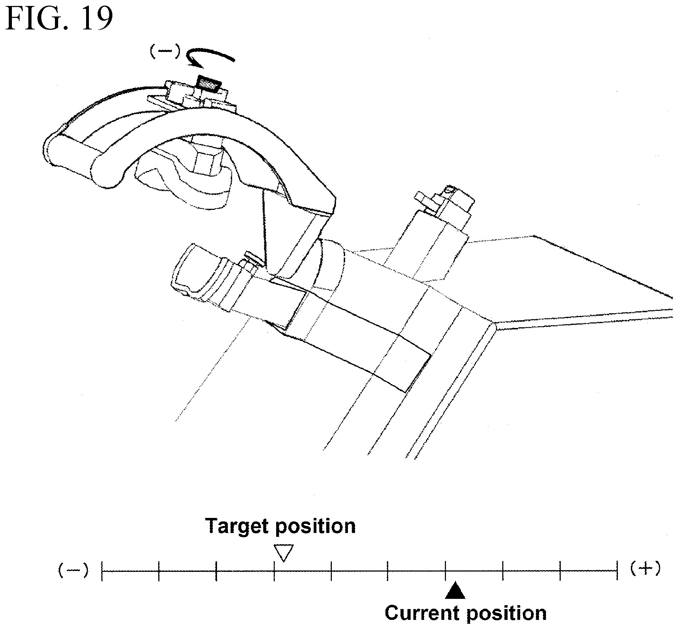

Also, the guidance section 502 displays the adjustment mechanism that is the subject of adjustment at the current point in time highlighted within the conceptual diagram of the positioning mechanical section 30, as well as displaying a direction for adjustment with arrows, as shown in Fig. 19. A linear indicator representing position of the movable member of the adjustment mechanism that is currently being adjusted is displayed, and marks showing target position and current position on the indicator are respectively displayed. Further, the guidance section 502 provides guidance for the adjustment mechanism that is the subject of adjustment at the current point in time, and adjustment direction, using voice. For example, guidance such as "please adjust adjustment mechanism number XX in the ?? direction" may be issued.

It should be noted that current position of the movable member of the adjustment mechanism is derived from position information that has been acquired by the acquisition section 504. Target position is derived by adding an amount to be adjusted, in a direction to be adjusted, to the current position before adjustment. The amount to be adjusted may also use an amount that is determined as a default (for example, a relative movement amount or target position that were determined beforehand for resolving one-sided contact), and may be determined based on some sort of information or conditions etc. (for example, determined automatically using arithmetic processing at the system side). It should be noted that highlighting may be realized by, for example, changing the color of the conceptual diagram of the adjustment mechanism that is the subject of adjustment, or the adjustment step, or by using half tone meshing.

In step T206, adjustment of an adjustment mechanism is commenced in accordance with guidance. Specifically, the operator 91 commences adjustment for an adjustment mechanism that is the subject of adjustment at the current point in time, while looking at an indicator, so that the current position coincides with the target position. The guidance section 502 updates display of marks showing the current position on the indicator substantially in real time.

In step T207, it is determined whether or not the current position and the target position are aligned. Specifically, the guidance section 502 determines whether or not the current position and target position of the adjustment mechanism are aligned. This alignment also includes not only complete alignment, but also alignment within a given permissible range. If the two positions are aligned, it is recognized that adjustment with the adjustment mechanism data that is the subject of adjustment at the current point in time has been completed, and processing advances to the next step. If alignment cannot be determined, it is possible to cause step T207 to be terminated in the next procedure. That is, when, in adjustment with the adjustment mechanism, target position cannot be accurately derived, for example, in cases such as where adjustment is performed according to physique, and position and shape of the head without feeling that something is wrong, after completion of adjustment the operator performs input of "adjustment complete" on the operation section 51. The guidance section 502 recognizes adjustment complete as a result of input of this "adjustment complete". If adjustment complete has been recognized, processing advances to the next step.

In step T208 it is determined whether or not there is an adjustment step to be performed next (that is, whether or not there is an adjustment mechanism that will be a subject of adjustment next). Specifically, the guidance section 502 determines whether or not there is an adjustment mechanism to be adjusted for the next sequence. If there is such an adjustment mechanism the adjustment mechanism for the next sequence is set to the adjustment mechanism that will be the subject of adjustment at the current point in time and processing returns to T205, while if there is not such an adjustment mechanism guidance is terminated.

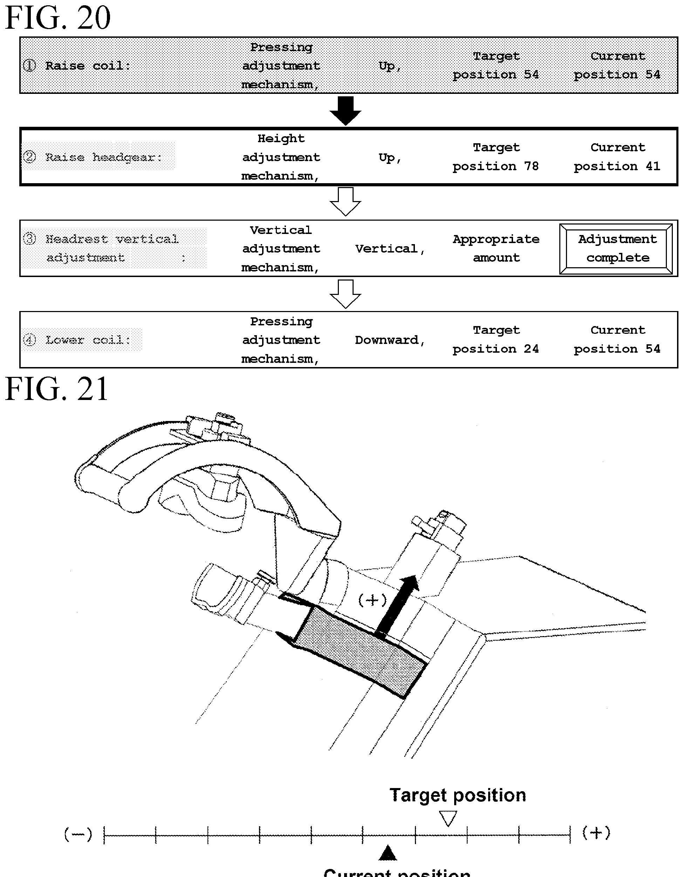

A display example of a case where guidance is performed for an adjustment procedure in order to fix a parietal region one-side contact state of the coil is shown in Fig. 20 and Fig. 21. This example is a case where an adjustment step that should be performed at the current point in time is the second adjustment step. In the flowchart representing the adjustment procedure, the first adjustment step is completed, and the second adjustment step is highlighted. Also, in the conceptual diagram of the positioning mechanical section 30 the adjustment mechanism to be adjusted in the second adjustment step is shown highlighted, and the adjustment direction for that adjustment mechanism is displayed using arrows in Fig. 21.

According to this second embodiment, it is possible to successively acquire position information for movable members of each adjustment mechanism, and more specific adjustment procedure guidance and guidance in accordance with the progress of adjustment become possible. Other structures and advantages of the second embodiment are the same as those of the previously described first embodiment, and so more detailed description has been omitted.

It should be noted that the position information that has been required may be transmitted, as required, to a device etc. that is different to the transcranial magnetic stimulation system 2, in a wired or wireless manner.

Also, a guidance section may be provided in a device or the like that is different to the control unit 50, and the control unit 50 may perform guidance for an adjustment procedure and movable member position that have been received from the guidance section.

The device etc. that is mentioned above is a server or a workstation, for example. The device etc. mentioned above may exist remotely from the transcranial magnetic stimulation system, or may exist in the vicinity.

### (First Modified Example)

A first modified example of the first and second embodiments will be described.

In provisional setting of the coil position, there may be cases where it is considered that the operator wishes to improve an adhered state of the coil section 11 to the subject's head.

Therefore, with this example, the receiving section 501 receives change for at least one of position and attitude of the coil section 11 such that a given (that is, defective) adhered state is improved, as the "desired change".

Also, with this example "a plurality of types of change" is change, for each of a plurality of types of inappropriate adhered state, for resolving that adhered state. A plurality of inappropriate adhered states are classified according to either a deficient contact state where even if position of the coil section 11 is adjusted in the pushing direction F there is not an appropriate position within the movement range of the coil section 11, and there is not sufficient adherence between the head contact surface of the coil section 11 and the subject's head, or an overly adhered state where the head contact surface of the coil section 11 is pressed excessively against the subject's head resulting in a feeling of discomfort for the subject. It should be noted that the classifications of adhered states are one example, and the present invention is not limited to these.

The adjustment procedure storage section 502A stores a plurality of items of adjustment procedure information respectively corresponding to a plurality of types of adhered state that have been registered beforehand.

The guidance section 502 reads out adjustment procedure information corresponding to adhered state that has been input, from the adjustment procedure storage section 502A, and performs guidance for the adjustment procedure represented by that adjustment procedure information.

According to this first modified example, the operator can fix an inappropriate adhered state of the coil by simply performing adjustment in accordance with an adjustment procedure for which guidance has been issued.

### (Second Modified Example)

A second modified example of the first and second embodiments will be described.

In provisional setting of the coil position, there may be cases where not only is it considered that the operator wishes to improve contact state and adhered state of the coil section 11 with the subject's head, but where it is considered that the operator wishes to change current position and attitude relatively by only a specified amount. This also includes deliberately bringing about a one-side contact state, according to circumstances.

Therefore, with this example, the receiving section 501 is input with change of a relative specific amount for at least one of position and attitude of the coil section 11. The receiving section 501 also receives the relative specific amount of change as the above described "desired change".

With this example, the above-described "relative specific amount of change" is a specific amount of movement in a specific direction, rotation by a specific rotational angle amount in a specific rotational direction, etc.

Also, with this example, the "plurality of types of change" are where combinations of movement direction and movement amount of the coil section 11 are respectively different, or where combinations of rotation direction and rotation angle amount are respectively different. As alternatives for movement direction and movement amount, it is possible to make movement direction, for example, forward, backward, left or right in the horizontal direction, up or down in the vertical direction, and to make movement amount 10 mm, 20 mm, or 30 mm. Also, as alternatives for rotation direction and rotation angle amount, it is possible to make rotation direction forward, backward, left, or right, and to make rotation angle amount 10°, 20°, or 30°.

The adjustment procedure storage section 502A stores adjustment procedure information in accordance with movement direction and movement amount that have been registered beforehand, and adjustment procedure information in accordance with rotation direction and rotation angle amount that have been registered beforehand.

The guidance section 502 reads out adjustment procedure information corresponding to movement direction and movement amount, or rotation direction and rotation angle amount, that have been input, from the adjustment procedure storage section 502A, and performs guidance for the adjustment procedure represented by that adjustment procedure information.

According to this second modified example, it is possible for the operator to move the coil section 11 in a desired direction and by a desired amount, or to rotate the coil section 11 in a desired rotation direction and by a desired angle amount, by simply performing adjustment in accordance with an adjustment procedure for which guidance has been issued.

### (Third Embodiment)

The transcranial magnetic stimulation system of the third embodiment has the same structure as the second embodiment, but the functions of the functional blocks in the control unit are different.

The receiving section 501 is input with at least one of position and attitude constituting targets for the coil section 11. That is, the receiving section 501 of this embodiment receives change in position and attitude of the coil section 11 such that the coil section 11 will attain target position and attitude, as "desired change". Here, the operator inputs desired position and attitude (orientation) in a coordinate system of the positioning mechanical section 30, and the receiving section 501 receives this input. It should be noted that input of desired position and attitude (orientation) for the coordinate system of the positioning mechanical section 30 are performed as follows, for example.

A three dimensional image that represents a virtual space in which it is possible to arrange the head of the subject 90 and the coil section 11 in the coordinate system at respectively arbitrary positions and attitudes, is displayed on the display section 52. The operator arranges the head and the coil section (or an image that models position and orientation at which magnetic stimulation is applied) within the virtual space, in an appropriate positional relationship for when treatment will actually be performed, on the basis of information relating to the subject that has been collected. Desired position and attitude of the coil section 11 are specified in the coordinate system of the positioning mechanical section 30, based on this positional relationship.

It should be noted that the three-dimensional image representing the head of the subject may be an image that has been modeled and not an actual image. Also, an image of the coil section may be an image resulting from having modeled position and orientation at which magnetic stimulation is applied.

The acquisition section 504 acquires position information of movable members of each adjustment mechanism that has been sent from the position information output section 60 by means of signals, as current position information. Acquisition of the current position information is repeatedly performed in comparatively short cycles, and successively updated.

The guidance section 502 obtains an adjustment procedure by computation based on current position information that has been acquired by the acquisition section 504, and target position and attitude of the coil section 11 that have been received by the receiving section 501, and provides guidance for this adjustment procedure. The adjustment procedure includes information as to which adjustment mechanism is adjusted, in which order, and to which position (on a scale).

Description will be given in the following of the processing flow for a case of performing guidance for an adjustment procedure for changing the coil section 11 to a target position and attitude.

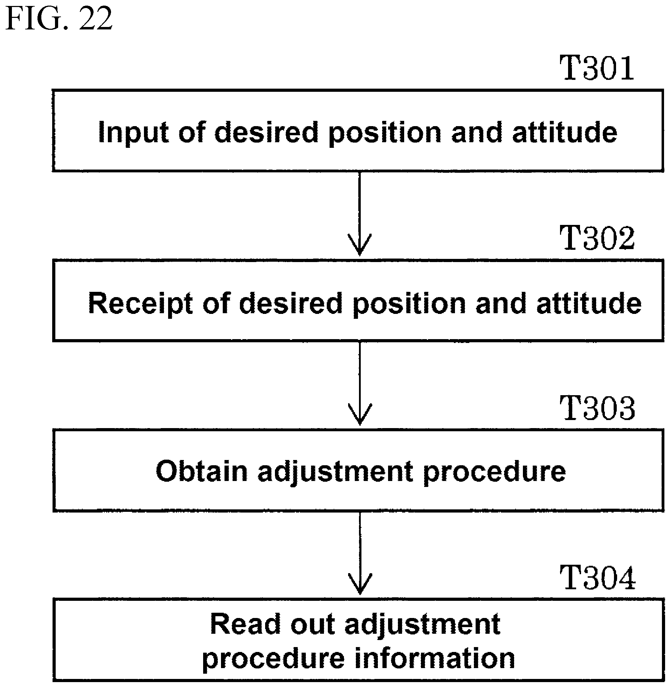

Fig. 22 is a flow chart showing flow of processing in a case where guidance is performed for an adjustment procedure in order to change the coil section 11 to a target position and attitude.

In step T301 desired position and attitude (orientation) are input. Specifically the operator inputs targets position and attitude for the coil section.

In step T302, input of the desired position and attitude is received. Specifically, the receiving section 501 receives that input of target position and attitude.

In step T303, an adjustment procedure is obtained. Specifically, the guidance section 502 obtains, by computation, an adjustment procedure for changing the coil section 11 from the current position and attitude to the target position and attitude that have been received.

In step T304, guidance for the adjustment procedure is provided. Specifically, the guidance section 502 provides guidance for the adjustment procedure that was obtained in step T302. Similarly to the second embodiment, together with displaying the adjustment procedure, current position of movable members of each adjustment mechanism is recognized, adjustment progress is monitored, and appropriate guidance is performed at each point in time.

### (Fourth Embodiment)

A transcranial magnetic stimulation system of the fourth embodiment is configured to automatically detect contact state of the coil section to the subject's head by quantitatively specifying a positional relationship of the coil section with respect to the subject's head using a sensor, and perform guidance of an adjustment procedure for the positioning mechanical section in order to improve or fix the contact state based on the results of the automatic detection.

It should be noted that the "contact state" mentioned above includes, for example, the adhered state (deficient contact, excessive contact etc.) and one-side contact state of the previously described embodiments.

The structure of the transcranial magnetic stimulation system of the fourth embodiment will be described.

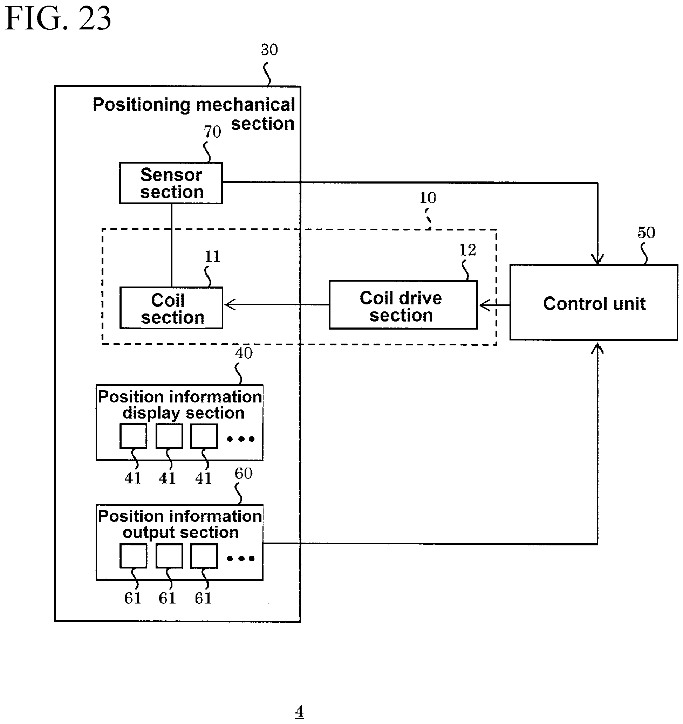

Fig. 23 is a drawing schematically showing the structure of a transcranial magnetic stimulation system 4 of the fourth embodiment.

As shown in Fig. 23, the transcranial magnetic stimulation system 4 comprises a magnetic stimulation circuit section 10, a positioning mechanical section 30, a position information display section 40, a position information output section 60, a sensor section 70, and a control unit 50. It should be noted that while, in Fig. 23, the sensor section 70 is included within the block representing the positioning mechanical section 30, this is because Fig. 23 is for showing an example where the sensor section 70 is physically close to the positioning mechanical section 30, and is not for defining an inclusion relation between these elements.

The magnetic stimulation circuit section 10, positioning mechanical section 30, position information display section 40, and position information output section 60 of this embodiment have the same structure as in the second embodiment. Also, similarly to the second embodiment, the magnetic stimulation circuit section 10 comprises a coil section 11 and a coil drive section 12.

The sensor section 70 includes one or more sensors arranged in the coil section 11 itself, or at a position where it is possible to overlook the coil section 11 and the subject's head, and outputs signals that reflect a positional relationship of the coil section 11 with respect to the subject's head. Details of the sensor section 70 will be described later.

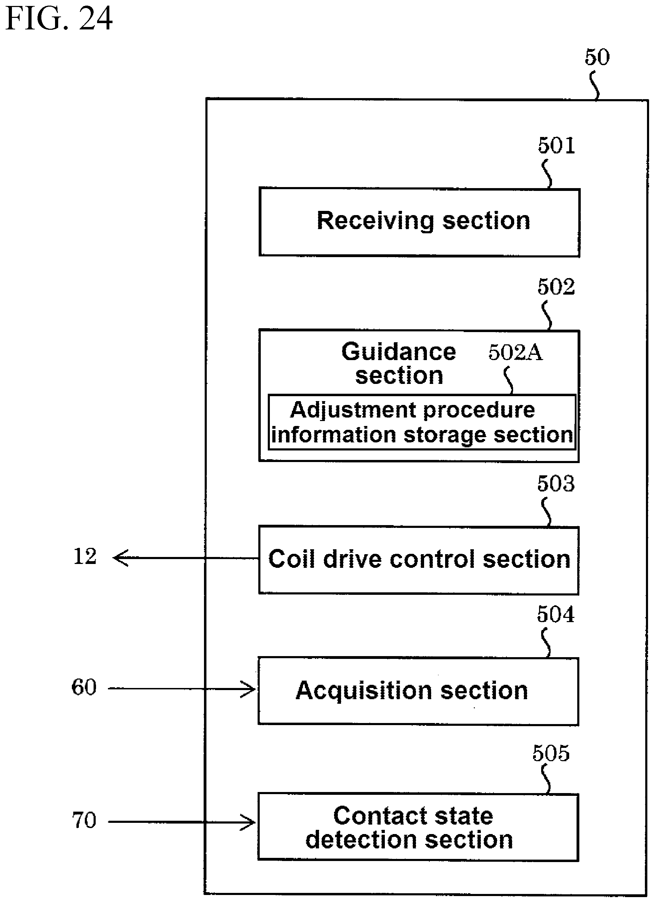

Fig. 24 is a functional block diagram of the control unit 50 of the fourth embodiment.

As shown in Fig. 24, the control unit 50 of this embodiment comprises a receiving section 501, guidance section 502, coil drive control section 503, acquisition section 504, and contact state detection section 505. It should be noted that the contact state detection section 505 is one example of detection means of the invention of this application.

The receiving section 501, guidance section 502, coil drive control section 503 and acquisition section 504 of this embodiment have at least the same functions as the second embodiment.

The contact state detection section 505 quantitatively specifies a positional relationship of the coil section 11 with respect to the subject's head based on signals that have been output from the sensor section 70, and detects adhered state and one-side contact state of the coil section 11. Specifically the contact state detection section 505 detects whether the coil section 11 is adhered to the subject's head at the proper pressure, whether one-side contact occurs, and in the case of one-side contact the extent to which the coil section 11 is offset and in which direction with respect to the head surface of the subject, or how inclined the coil section 11 is, etc., is detected with high resolution based on output signals from the sensor section 70.

The sensor section 70 includes one or more, and one or more types, of sensor from among, for example, an imaging sensor, an ultrasonic sensor, an infrared sensor, a pressure sensor, a mechanical switch sensor, an electrostatic sensor, and an acceleration sensor etc.

For example, the sensor section 70 may be configured with one or a plurality of imaging sensors fitted close to the head contact surface of the coil section 11 or at a position where the coil section 11 and the subject's head can be overlooked. In this case, the imaging sensors form images of either the subject's head, or both the coil section 11 and the subject's head, and output image signals that have been acquired. The contact state detection section 505 executes analysis processing based on the image signals, and performs image recognition for the subject's head or both the coil section 11 and the subject's head, and detects a one-side contact state of the coil section 11 with respect to the head surface from the results of this detection.

Also, for example, the sensor section 70 may be configured having a plurality of ultrasonic sensors fitted on the head contact surface of the coil section 11, close to the head contact surface, or at an outer edge section of the head contact surface etc. In this case, each ultrasonic sensor respectively outputs signals corresponding to a distance from the sensor to the subject's head. The contact state detection section 505 executes analysis processing based on the signals, recognizes a distance from a position of each sensor to the head surface of the subject, and detects one-side contact state of the coil section 11 with respect to the head surface from the results of this detection.

Also, for example, the sensor section 70 may be configured having a plurality of infra-red sensors fitted on the head contact surface of the coil section 11, close to the head contact surface, or at an outer edge section of the head contact surface etc. In this case, each infrared sensor respectively outputs signals corresponding to a distance from the sensor to the subject's head. The contact state detection section 505 executes analysis processing based on the signals, recognizes a distance from a position of each sensor to the head surface of the subject, and detects one-side contact state of the coil section 11 with respect to the head surface from the results of this detection.

Also, for example, the sensor section 70 may be configured having a plurality of pressure sensors fitted on the head contact surface of the coil section 11, or at an outer edge section of the head contact surface. In this case, each pressure sensor respectively outputs a signal corresponding to pressure that has been applied from the head in making contact with the subject's head. The contact state detection section 505 executes analysis processing based on the signals. In this way, for each sensor position, the contact state detection section 505 recognizes whether there is contact with the subject's head or, if there is contact, to what extent contact is adhered, and detects adhered state and one-side contact state of the coil section 11 with respect to head surface from the results of this recognition.

Also, for example, the sensor section 70 may be configured having a plurality of mechanical switch sensors fitted on the head contact surface of the coil section 11, or at an outer edge section of the head contact surface. In this case, each mechanical switch sensor is normally held off, but is turned on if a switch section contacts the subject's head and is pressed by a specified amount with a given pressure or more, and respectively outputs a signal representing this on/off state. The contact state detection section 505 executes analysis processing based on these signals. In this way, for each sensor position, the contact state detection section 505 recognizes whether or not there is contact with the subject's head, and detects one-side contact state of the coil section 11 with respect to head surface from the results of this detection. It should be noted that the mechanical switch sensors can acquire states at a plurality of stages in response to an amount by which the switch section is pressed in, and in a case where the mechanical switch sensors are configured so as to output signals representing the states, it is also possible to detect adhered state of the coil section 11 with respect to the head surface.

Also, for example, the sensor section 70 may be configured having a plurality of electrostatic sensors fitted on the head contact surface of the coil section 11, or at an outer edge section of the head contact surface. In this case, each electrostatic sensor respectively outputs signals corresponding to distance from the sensor to the subject's head, or signals representing whether or not the sensor is in contact with the subject's head. The contact state detection section 505 executes analysis processing based on these signals. In this way, for each sensor position, the contact state detection section 505 recognizes distance to the subject's head and whether or not there is contact with the subject's head, and detects one-side contact state of the coil section 11 with respect to head surface from the results of this detection.

Also, for example, the sensor section 70 may be configured having an acceleration sensor further fitted to the coil section 11, in addition to the above-described sensors. In this case, the acceleration sensor outputs signals representing respective components of gravitational acceleration applied to the sensor, in the rectangular coordinate system of the coil section 11. The contact state detection section 505 executes analysis processing based on these signals. In this way, the contact state detection section 505 recognizes inclination of the coil section 11, and detects one-side contact state with respect to the subject's head with good precision by supplementing information relating to position an attitude of the coil section 11 that has been acquired by other sensors, from the results of this recognition.

Also, for example, the sensor section 70 may be configured with combinations of the above-described sensors.

As stated above, if the contact state detection section 505 detects inappropriate adhered state or one-side contact state of the coil section 11 based on signals from the sensor section 70, the receiving section 501 receives input of the states, and the guidance section 502 obtains an adjustment procedure for improving that adhered state or resolving that one-side contact state, and issues guidance to the operator.

It should be noted, regarding the way in which the adjustment procedure is obtained at this time, that is, regarding operation of the receiving section 501, guidance section 502 and contact state detection section 505 of the control unit 50, that the following three cases can be considered, for example.

With a first case, the contact state detection section 505 inputs data that quantitatively represents contact state of the coil section 11, that is, adhered state and one-side contact state, to the receiving section 501. In this case, the guidance section 502 obtains adjustment direction and adjustment amount (movement amount), or target position (for example, a target value for a numerical value displayed by the position information display section 40) for each adjustment mechanism by mathematical analysis, using a method such as three dimensional analysis that takes into consideration the degree of freedom of each adjustment mechanism, based on data that has been input to the receiving section 501. It should be noted that while the guidance section 502 normally obtains adjustment amount or target position by computation, these may be obtained using a previously prepared correspondence table.

With a second case, the contact state detection section 505 specifies type of adhered state and one-side contact state of the coil section 11 from data that quantitatively represents contact state of the coil section 11, that is, adhered state and one-side contact state, and inputs this to the receiving section 501. In this case, in accordance with a type of adhered state or one-side contact state that has been input to the receiving section 501, the guidance section 502 obtains a corresponding adjustment procedure using computation or a correspondence table.

With a third case, the contact state detection section 505 reports data that quantitatively represents contact state of the coil section 11, that is, adhered state and one-side contact state, or type of adhered state or one-side contact state of the coil section 11 specified from that data, to the operator. The operator inputs type of adhered state or one-side contact state of the coil section 11 to the receiving section 501 on the basis of this information that has been reported. Input of the type of adhered state or one-side contact state is performed by the operator selecting one from a plurality of alternatives that have been prepared beforehand, similarly to the second embodiment, for example. The guidance section 502 obtains an adjustment procedure for improving the type of adhered state or one-side contact state that has been input using computation or a correspondence table.

It should be noted that the guidance method for the adjustment procedure by the guidance section 502 can be considered to be the same method as for the previously described embodiments.

According to this fourth embodiment, the contact state detection section 505 detects contact state of the coil section 11 with respect to the head using the sensor section 70, and the receiving section 501 receives input of the contact state that has been detected, since it is then possible to detect contact state of the coil section 11 with the head, particularly adhered state and one-side contact state, automatically, and guidance for an adjustment procedure can be provided on the basis of the results of this detection. Therefore, the operator can receive guidance for the adjustment procedure after having specified the contact state without relying on experience and intuition.

Also, in a case where the receiving section 501 automatically receives input of the contact state that has been detected, it is possible to receive guidance for an adjustment procedure without the involvement of an operation by the operator themselves, and it is possible to reduce the burden on the user due to decisions and operations.

Also, in a case where the contact state detection section 505 quantitatively detects the contact state and the guidance section 502 obtains adjustment amounts or target position for an adjustment mechanism based on that contact state that has been quantitatively detected, the operator can expect guidance to be issued for a higher accuracy adjustment procedure.

While embodiments of the invention have been described above, embodiments of the present invention are not limited to these and various changes are possible within a scope that does not depart from the gist of the present invention.

For example, the operation section 51 and the display section 52 may be a mobile terminal that has a touch panel. By doing this it is possible for the operator to perform adjustment of an adjustment mechanism while referencing an adjustment procedure that they have to hand, which is convenient.

Also, for example, the transcranial magnetic stimulation system is not limited to being used with pain relief treatment, and may also be used in the treatment of depression and research.

Also, for example, the "desired change" for the coil section may be change of at least one of position and attitude of the coil section.

Also, for example, the positioning mechanical section may have any structure as long as control is performed so as to move the coil section along a curve that corresponds to the head surface of a subject, and may be something like a robot arm.

### Description Of The Numerals

- 1, 2: transcranial magnetic stimulation system
- 10: magnetic stimulation circuit section
- 11: coil section
- 12: coil drive section
- 30: positioning mechanical section
- 31: chair
- 32: physique adjustment mechanism
- 321: occipital region rest
- 322: forward and backward direction adjustment mechanism
- 323: vertical direction adjustment mechanism
- 324: physique adjustment guide
- 325: height adjustment mechanism
- 33: headgear
- 331: pushing direction adjustment mechanism
- 332: yaw direction adjustment mechanism
- 333: pitch direction adjustment mechanism
- 334: roll direction adjustment mechanism
- 335: headgear guide
- 336: attaching/detaching mechanism
- 40: position information display section
- 41: position display section
- 50: control unit
- 51: operation section
- 52: display section
- 53: computation section
- 54: interface section
- 55: storage section
- 60: position information output section
- 61: position detection section
- 70: sensor section
- 90: subject
- 501: receiving section
- 502: guidance section
- 503: coil drive control section
- 504: acquisition section
- 505: contact state detection section

## Claims

1. A transcranial magnetic stimulation system, comprising:
a coil section that comes in to contact with or approaches a head of a subject;
a mechanical section, comprising a plurality of adjustment mechanisms that adjust position of a plurality of movable members, that determines position and attitude of the coil section by adjustment using the plurality of adjustment mechanisms;
receiving means that receives desired change for at least one of position and attitude of the coil section; and
guidance means that provides guidance of an adjustment procedure for at least one of the plurality of adjustment mechanisms so as to realize the desired change that has been received.

2. The transcranial magnetic stimulation system of claim 1, wherein the receiving means receives input of contact state of the coil section with the head, as the desired change.

3. The transcranial magnetic stimulation system of claim 2, wherein the contact state is a one-side contact state where only a part, of an outer edge section of the coil section, contacts the head.

4. The transcranial magnetic stimulation system of claim 3, wherein the receiving means has choice of a plurality of types of one-side contact state having respectively different state of contact of the outer edge section with the head, and a single one-side contact state selected by an operator is input as the contact state.

5. The transcranial magnetic stimulation system of the claim 2, where contact state includes an adhered state of the coil section to the head.

6. The transcranial magnetic stimulation system of claim 1, wherein the receiving means receives a specified amount of change for at least one of position and attitude of the coil section as the desired change.

7. The transcranial magnetic stimulation system of claim 6, wherein the relative specified amount of change is specified amount of movement in a specified direction, or rotation through a specified angular amount for a specified rotational direction.

8. The transcranial magnetic stimulation system of claim 7, wherein the receiving means has choice of a plurality of respectively different types of change that differ in terms of combination of the specified direction and given movement amount, or combination of the specified rotation direction and angular amount, and one change is selected by the operator as the desired change.

9. The transcranial magnetic stimulation system of any one of claim 1 to claim 8, further comprising acquisition means that acquires position information representing position of the plurality of movable members at the current time, and wherein the guidance means performs guidance of the adjustment procedure using position information that has been acquired by the acquisition means.

10. The transcranial magnetic stimulation system of claim 1, further comprising acquisition means that acquires position information representing position of the plurality of movable members at the current time, and wherein the receiving means receives at least one of position and attitude, constituting targets for the coil section, as the desired change.

11. The transcranial magnetic stimulation system of claim 10, wherein the guidance means performs guidance of the adjustment procedure using position information that has been acquired by the acquisition means.

12. The transcranial magnetic stimulation system of claim 11, wherein the guidance means determines whether or not position represented by the position information that has been acquired coincides with a position that will be guided using the adjustment procedure, or coincides with being within a permissible range.

13. The transcranial magnetic stimulation system of any one of claim 1 to claim 12, wherein the guidance means shows at least order in which the adjustment mechanisms should be adjusted, and adjustment direction of the adjustment mechanisms.

14. The transcranial magnetic stimulation system of any one of claim 1 to claim 13, wherein the guidance means displays an image that emphasizes or indicates the adjustment mechanisms or the movable members that are to be adjusted at the current point in time.

15. The transcranial magnetic stimulation system of any one of claim 1 to claim 14, wherein the guidance means provides guidance of the adjustment procedure using sound.

16. The transcranial magnetic stimulation system of any one of claim 1 to claim 15, wherein the mechanical section is controlled so that the coil section moves along a curved line corresponding to the surface of the head of a subject, as one movement direction.

17. The transcranial magnetic stimulation system of any one of claim 1 to claim 16, wherein the mechanical section is controlled so that the coil section moves in a vertical direction of a subject, as one movement direction.

18. The transcranial magnetic stimulation system of any one of claim 1 to claim 17, wherein the mechanical section is controlled so that the coil section moves along a direction towards substantially the center of the head of a subject, as one movement direction.

19. A positioning assistance method for a mechanical section of a transcranial magnetic stimulation system, wherein,
the mechanical section comprises a plurality of adjustment mechanisms that adjust position of a plurality of movable members, and determines position and attitude of the coil section by adjustment using the plurality of adjustment mechanisms, and wherein the method causes a device to execute:
a receiving process for receiving desired change for at least one of position and attitude of the coil section, and
a guidance process for providing guidance of an adjustment procedure for at least one of the plurality of adjustment mechanisms so as to achieve the desired change that has been received.

20. A computer program for a positioning assistance method for a mechanical section of a transcranial magnetic stimulation system, wherein
the mechanical section comprises a plurality of adjustment mechanisms that adjust position of a plurality of movable members, and determines position and attitude of the coil section by adjustment using the plurality of adjustment mechanisms, and the computer program causes a computer to execute:
a receiving process for receiving desired change for at least one of position and attitude of the coil section, and
a guidance process for providing guidance of an adjustment procedure for at least one of the plurality of adjustment mechanisms so as to achieve the desired change that has been received.

21. The transcranial magnetic stimulation system of claim 2, further comprising detection means that detects contact state of the coil section with respect to the head, using a sensor section that includes one or more sensors, and wherein
the receiving means receives input of contact state that has been detected.

22. The transcranial magnetic stimulation system of claim 21, wherein the detection means quantitatively detects the contact state, and
the guidance means obtains adjustment amount or target position for the adjustment mechanism based on contact state that has been quantitatively detected.

23. The transcranial magnetic stimulation system of claim 21 or claim 22, wherein the contact state includes at least one of a one-side contact state where only part of an outer edge section of the coil section contacts the head, and an adhered state where the coil section contacts the head.

24. The transcranial magnetic stimulation system of any one of claim 21 to claim 24, wherein the sensor section includes at least one among an imaging sensor, an ultrasonic sensor, an infra-red sensor, a pressure sensor, a mechanical switch sensor, an electrostatic sensor, and an acceleration sensor.
